# EUROPEAN PATENT APPLICATION

(11) **EP 4 825 380 A1**
(43) Date of publication of application: **21.10.2026**
(21) Application number: 24903761.5
(22) Date of filing: 12.12.2024
(51) Int. Cl.: A61K 31/519, A61P 35/00, A61P 35/04, A61P 43/00

(54) **BRAIN-MIGRATING TUMOR TREATMENT AGENT CONTAINING, AS ACTIVE INGREDIENT, N-(4-(4-AMINO-6-ETHYNYL-5-(QUINOLIN-3-YL)-7H-PYRROLO[2,3-D]PYRIMIDIN-7-YL) BICYCLO[2.2.1]HEPTAN-1-YL)-5-METHYLPYRAZINE-2-CARBOXAMIDE OR SALT THEREOF**

(30) Priority: 13.12.2023 JP 2023210446; 30.09.2024 JP 2024170941
(71) Applicant: Taiho Pharmaceutical Co., Ltd., Chiyoda-ku, Tokyo 101-8444 (JP)
(72) Inventor: KASUGA Hidefumi, Tsukuba-shi, Ibaraki 300-2611 (JP)
(74) Representative: Wächtershäuser & Hartz Patentanwaltspartnerschaft mbB
(86) International application number: PCT/JP2024/044041
(87) International publication number: WO 2025/127108

(57) **Abstract**

The present invention provides a brain-migrating anti-tumor agent that exhibits brain migration ability and EGFR inhibitory activity. One embodiment of the present invention provides a brain-migrating anti-tumor agent comprising, as an active ingredient, N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof.

## Description

### TECHNICAL FIELD

The present invention relates to a brain-penetrable antitumor agent or the like comprising a compound having an inhibitory effect against the epidermal growth factor receptor (EGFR).

### BACKGROUND ART

Brain tumor is one of the brain diseases and is a generic name for tumors occurring intracranially. Various types of tumors develop from each site, and these tumors are divided into two groups, i.e., primary brain tumors and metastatic brain tumors. Primary brain tumors refer to those developing from cells or nerves in the brain and membranes covering the brain, while metastatic brain tumors refer to those caused when cancers such as lung cancer and breast cancer metastasize through the bloodstream to the brain. Symptomatic metastatic brain tumors are reported to occur in 8% to 10% of cancer patients, and lung cancer patients are reported to have brain metastasis with a frequency of 40% to 50%, as assessed by autopsy (Non Patent Literatures 1 to 3). Examples known as primary lesions of metastatic brain tumors include lung cancer, breast cancer, digestive system cancer (gastric cancer), malignant melanoma, and the renal and urogenital system. Among them, lung cancer accounts for almost half of all cases (Non Patent Literature 2). Metastatic brain tumors require different therapies depending on the patient's general condition, the size of tumor, the number of metastatic sites, etc. Therapies for this purpose include radiotherapy, surgery, drug therapy, or combinations thereof. In general, surgical operation may be selected, e.g., for patients whose primary lesion is controlled and solitary and who can be expected to survive for some period of time. However, tumors having metastasized into the brain are often complexly intermingled with the brain parenchyma and/or brain tissue, and surgical operation is not sufficient in many cases to completely excise only tumors. Radiotherapy is known to have two types, i.e., stereotactic radiosurgery (e.g., gamma knife therapy), and whole brain radiation therapy designed to irradiate the whole brain. Current treatment of metastatic brain tumors is mostly based on surgical operation combined with radiotherapy, but it is hard to be regarded as exerting a sufficient therapeutic effect (Non Patent Literatures 4 and 5). In general, the central nervous system (CNS) including the brain is protected by a tight junction layer of highly specialized cells, which is referred to as the blood brain barrier (BBB), in such a way as to prevent the entry of harmful substances. One of the reasons that no effective drug has been developed against most of CNS-related diseases is because almost all therapeutic molecules including antibodies are unable to pass through the BBB. Small molecule compounds are also not excepted, and there is a report showing that more than 97% of small molecule compounds are unable to pass through the BBB (Non Patent Literature 6). At present, there are many compounds effective against peripheral cancers (primary lesions), but they have limited efficacy in brain metastatic lesions of these cancers. One of the reasons for such limited efficacy is that these compounds do not reach the brain in sufficient amounts to exert their effect, and therefore do not exert the same effect as in primary lesions (Non Patent Literatures 7 and 8). For these reasons, in CNS-related diseases and brain tumors, there has been a demand for the development of small molecule compounds with high brain penetration and a methodology for their brain penetration. Particularly in the treatment of metastatic brain tumors, there has been a demand for drugs showing an effect which is also effective against primary lesions (e.g., lung cancer) and showing high CNS penetration, and also showing an effect against brain metastatic lesions.

There are many compounds effective against primary lesions, as exemplified by compounds having inhibitory activity against the epidermal growth factor receptor (EGFR). EGFR is a receptor-type tyrosine kinase and exerts its physiological functions in normal tissue upon binding to its ligand, i.e., epidermal growth factor (EGF). In the epidermal tissue, EGFR contributes to growth and apoptosis inhibition, etc. (Non Patent Literature 9). Moreover, EGFR is also a kind of oncogene, and amplification of the EGFR gene and high expression or mutation of its protein have been known in various cancer types including head and neck cancer, breast cancer, large bowel cancer, esophageal cancer, pancreatic cancer, lung cancer, ovarian cancer, renal cancer, bladder cancer, skin cancer, brain tumor and so on (Non Patent Literature 10).

Thus, EGFR inhibitors are deemed to be useful as therapeutic agents for diseases related to abnormal hyperactivation of the above pathways.

Actually, medicaments such as erlotinib, gefitinib, afatinib and osimertinib are used as EGFR inhibitors in the clinical setting, and contribute to improvements in treatment outcomes. However, among these drugs, some drugs have a problem in that they cause non-selective inhibition of wild-type EGFR (WT), thus leading to the development of side effects. Since brain tumor is mostly metastasized from lung cancer, studies have also been made on the brain penetration of these EGFR inhibitors for use in the treatment of lung cancer. As a result, it has been elucidated that these EGFR inhibitors are not all able to pass through the BBB (Non Patent Literatures 11, 12 and 13).

Patent Document 1 discloses, as an EGFR-inhibiting compound, a pyrimidine compound represented by the following general formula (1): 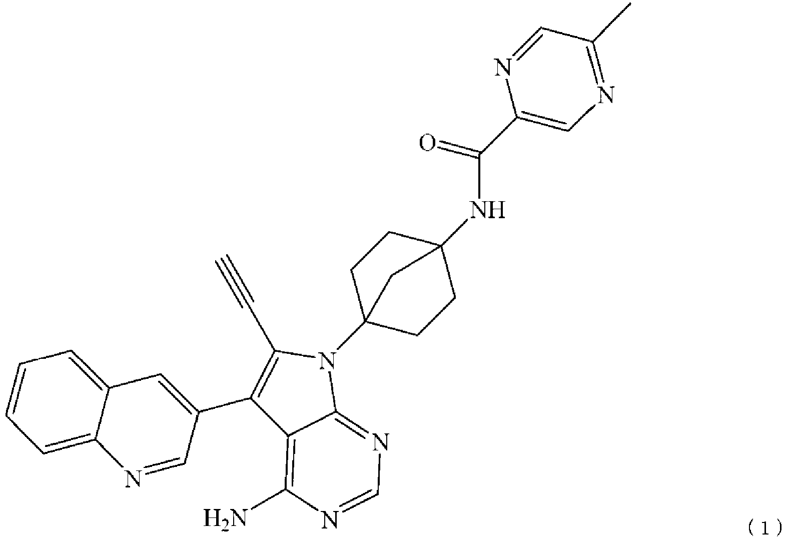 (chemical name: N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide) (hereinafter also referred to as "compound (1)").

### CITATION LIST

### Patent Literature

Patent Literature 1 : WO2020/166680

### Non Patent Literature

Non Patent Literature 1: Qingbei Zeng et al., J. Med. Chem.; 58(20): p. 8200-8215 (2015)
Non Patent Literature 2: Lakshmi Nayak et al., Curr Oncol Rep; 14(1): p. 48-54 (2012)
Non Patent Literature 3: Brunilde Gril et al., Eur. J. Cancer; 46(7): p. 1204-1210 (2010)
Non Patent Literature 4: Taofeek K. Owonikoko et al., Nat. Rev. Clin. Oncol.; 11(4): p. 203-222 (2014)
Non Patent Literature 5: Chien-Hung Gow et al., Clin Cancer Res; 14(1): p. 162-168 (2008)
Non Patent Literature 6: William M. Pardridge et al., J. Neurochem.; 70(5): p. 1781-1792 (1998)
Non Patent Literature 7: Ted W. Johnson et al., J. Med. Chem.; 57(11): p. 4720-4744 (2004)
Non Patent Literature 8: Victor A. Levin et al., Neuro Oncology; 17 Suppl 6: vi1-26 (2015)
Non Patent Literature 9: Mario E. Lacouture, Nature Rev. Cancer; 6: p. 803-812 (2006)
Non Patent Literature 10: Ping Wee et al., Cancers (Basel); 9(5); 52: p. 1-45 (2017)
Non Patent Literature 11: Chee-Seng Tan et al., Lung Cancer; 108: p. 29-37 (2017)
Non Patent Literature 12: Nicola Colclough et al., Clin Cancer Res; 27(1): p. 189-201 (2021)
Non Patent Literature 13: Peter Ballard et al., Clin Cancer Res; 22(20): p. 5130-5140 (2016)
Non Patent Literature 14: Giacomini KM. et al., Nat Rev Drug Discov; 9: pp. 215-236, (2010)

### SUMMARY OF THE INVENTION

### PROBLEM TO BE SOLVED BY THE INVENTION

Under circumstances as mentioned above, there has been a demand for a brain-penetrable antitumor agent showing brain penetration and EGFR inhibitory activity.

### MEANS TO SOLVE THE PROBLEM

As a result of extensive and intensive efforts, the inventors of the present invention have found that a pyrimidine compound of particular structure selectively inhibiting mutant EGFR (chemical name: N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide; hereinafter also referred to as compound (1)) or a salt thereof is useful as a brain-penetrable antitumor agent, and completed the present invention.

Namely, the present invention includes the following embodiments.

[1] A brain-penetrable antitumor agent, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof as an active ingredient.
[2] The brain-penetrable antitumor agent according to [1] above, for the treatment of primary or metastatic brain tumor.
[3] The brain-penetrable antitumor agent according to [2] above, wherein the brain tumor has an EGFR mutation of C797X, where X represents any amino acid.
[3-1] The brain-penetrable antitumor agent according to [2] above, wherein the brain tumor has an EGFR mutation of Ex19del.
[3-2] The brain-penetrable antitumor agent according to [2] above, wherein the brain tumor has an EGFR mutation of L858R.
[4] The brain-penetrable antitumor agent according to [3] above, wherein C797X is C797S or C797G.
[5] The brain-penetrable antitumor agent according to [4] above, wherein the brain tumor further has an EGFR mutation of Ex19del, preferably Del E746-A750.
[5-1] The brain-penetrable antitumor agent according to [4] above, wherein the brain tumor further has an EGFR mutation of L858R.
[6] The brain-penetrable antitumor agent according to [5] or [5-1] above, wherein the brain tumor further has an EGFR mutation of T790M.
[7] The brain-penetrable antitumor agent according to any one of [1] to [6] and [5-1] above, for administration to a subject who has been treated with osimertinib.
[8] A therapeutic agent for brain tumor, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof as an active ingredient.
[9] The therapeutic agent for brain tumor according to [8] above, for the treatment of primary or metastatic brain tumor.
[10] The therapeutic agent for brain tumor according to [9] above, wherein the brain tumor has an EGFR mutation of C797X, where X represents any amino acid.
[10-1] The therapeutic agent for brain tumor according to [9] above, wherein the brain tumor has an EGFR mutation of Ex19del.
[10-2] The therapeutic agent for brain tumor according to [9] above, wherein the brain tumor has an EGFR mutation of L858R.
[11] The therapeutic agent for brain tumor according to [10] above, wherein C797X is C797S or C797G.
[12] The therapeutic agent for brain tumor according to [11] above, wherein the brain tumor further has an EGFR mutation of Ex19del, preferably Del E746-A750.
[12-1] The therapeutic agent for brain tumor according to [11] above, wherein the brain tumor further has an EGFR mutation of L858R.
[13] The therapeutic agent for brain tumor according to [12] or [12-1] above, wherein the brain tumor further has an EGFR mutation of T790M.
[14] The therapeutic agent for brain tumor according to any one of [8] to [13] and [12-1] above, for administration to a subject who has been treated with osimertinib.
[15] N-(4-(4-Amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof for use in the treatment of brain tumor.
[16] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [15] above, for the treatment of primary or metastatic brain tumor.
[17] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [16] above, wherein the brain tumor has an EGFR mutation of C797X, where X represents any amino acid.
[17-1] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [16] above, wherein the brain tumor has an EGFR mutation of Ex19del.
[17-2] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [16] above, wherein the brain tumor has an EGFR mutation of L858R.
[18] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [17] above, wherein C797X is C797S or C797G.
[19] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [18] above, wherein the brain tumor further has an EGFR mutation of Ex19del, preferably Del E746-A750.
[19-1] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [18] above, wherein the brain tumor further has an EGFR mutation of L858R.
[20] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to [19] or [19-1] above, wherein the brain tumor further has an EGFR mutation of T790M.
[21] The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to any one of [15] to [20] and [19-1] above, for administration to a subject who has been treated with osimertinib.
[22] The use of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof for the manufacture of a medicament for the treatment of brain tumor.
[23] The use according to [22] above, for the treatment of primary or metastatic brain tumor.
[24] The use according to [23] above, wherein the brain tumor has an EGFR mutation of C797X, where X represents any amino acid.
[24-1] The use according to [23] above, wherein the brain tumor has an EGFR mutation of Ex19del.
[24-2] The use according to [23] above, wherein the brain tumor has an EGFR mutation of L858R.
[25] The use according to [24] above, wherein C797X is C797S or C797G.
[26] The use according to [25] above, wherein the brain tumor further has an EGFR mutation of Ex19del, preferably Del E746-A750.
[26-1] The use according to [25] above, wherein the brain tumor further has an EGFR mutation of L858R.
[27] The use according to [26] or [26-1] above, wherein the brain tumor further has an EGFR mutation of T790M.
[28] The use according to any one of [22] to [27] and [26-1] above, for administration to a subject who has been treated with osimertinib.
[29] The use of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof for the treatment of brain tumor.
[30] A method for treating a subject with brain tumor, comprising administering N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof to a subject in need of treatment.
[31] The method for treating according to [30] above, for the treatment of primary or metastatic brain tumor.
[32] The method for treating according to [31] above, wherein the brain tumor has an EGFR mutation of C797X, where X represents any amino acid.
[32-1] The method for treating according to [31] above, wherein the brain tumor has an EGFR mutation of Ex19del.
[32-2] The method for treating according to [31] above, wherein the brain tumor has an EGFR mutation of L858R.
[33] The method for treating according to [32] above, wherein C797X is C797S or C797G.
[34] The method for treating according to [33] above, wherein the brain tumor further has an EGFR mutation of Ex19del, preferably Del E746-A750.
[34-1] The method for treating according to [33] above, wherein the brain tumor further has an EGFR mutation of L858R.
[35] The method for treating according to [34] or [34-1] above, wherein the brain tumor further has an EGFR mutation of T790M.
[36] The method for treating according to any one of [30] to [35] and [34-1] above, for administration to a subject who has been treated with osimertinib.
[37] A pharmaceutical composition for the treatment of brain tumor, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof and a pharmaceutically acceptable carrier.
[38] An anti-brain tumor agent, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof as an active ingredient.
[39] The anti-brain tumor agent according to [38] above, for the treatment of primary or metastatic brain tumor.
[40] The anti-brain tumor agent according to [39] above, wherein the brain tumor has an EGFR mutation of C797X, where X represents any amino acid.
[40-1] The anti-brain tumor agent according to [39] above, wherein the brain tumor has an EGFR mutation of Ex19del.
[40-2] The anti-brain tumor agent according to [39] above, wherein the brain tumor has an EGFR mutation of L858R.
[41] The anti-brain tumor agent according to [40] above, wherein C797X is C797S or C797G.
[42] The anti-brain tumor agent according to [41] above, wherein the brain tumor further has an EGFR mutation of Ex19del, preferably Del E746-A750.
[42-1] The anti-brain tumor agent according to [41] above, wherein the brain tumor further has an EGFR mutation of L858R.
[43] The anti-brain tumor agent according to [42] or [42-1] above, wherein the brain tumor further has an EGFR mutation of T790M.
[44] The anti-brain tumor agent according to any one of [38] to [43] and [42-1] above, for administration to a subject who has been treated with osimertinib.
[45] A brain-penetrable mutant EGFR inhibitor, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof as an active ingredient.
[46] The brain-penetrable mutant EGFR inhibitor according to [45] above, for the treatment of primary or metastatic brain tumor.
[47] The brain-penetrable mutant EGFR inhibitor according to [46] above, wherein the brain tumor has an EGFR mutation of C797X, where X represents any amino acid.
[47-1] The brain-penetrable mutant EGFR inhibitor according to [46] above, wherein the brain tumor has an EGFR mutation of Ex19del.
[47-2] The brain-penetrable mutant EGFR inhibitor according to [46] above, wherein the brain tumor has an EGFR mutation of L858R.
[48] The brain-penetrable mutant EGFR inhibitor according to [47] above, wherein C797X is C797S or C797G.
[49] The brain-penetrable mutant EGFR inhibitor according to [48] above, wherein the brain tumor further has an EGFR mutation of Ex19del, preferably Del E746-A750.
[49-1] The brain-penetrable mutant EGFR inhibitor according to [48] above, wherein the brain tumor further has an EGFR mutation of L858R.
[50] The brain-penetrable mutant EGFR inhibitor according to [49] or [49-1] above, wherein the brain tumor further has an EGFR mutation of T790M.
[51] The brain-penetrable mutant EGFR inhibitor according to any one of [45] to [50] and [49-1] above, for administration to a subject who has been treated with osimertinib.

### EFFECTS OF THE INVENTION

According to one aspect of the present invention, there is provided a brain-penetrable antitumor agent. Moreover, according to another aspect of the present invention, there is provided an anti-brain tumor agent. Furthermore, according to yet another aspect of the present invention, there is provided a therapeutic agent for brain tumor or a brain-penetrable mutant EGFR inhibitor.

### BRIEF DESCRIPTION OF DRAWINGS

Figure 1 shows the results (luminescence level) of the efficacy evaluation test of compound (1) and brigatinib in a mouse brain transplantation model in Test Example 4.
Figure 2 shows the results (survival curve) of the efficacy evaluation test of compound (1) and brigatinib in a mouse brain transplantation model in Test Example 4.
Figure 3 shows the results of the efficacy evaluation test of compound (1) in a mouse subcutaneous transplantation model in Test Example 5.
Figure 4 shows the results of the efficacy evaluation test of brigatinib in a mouse subcutaneous transplantation model in Test Example 6.

### DESCRIPTION OF EMBODIMENTS

One aspect of the present invention relates to a brain-penetrable antitumor agent, which comprises N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide represented by the following formula (1) or a salt thereof as an active ingredient.

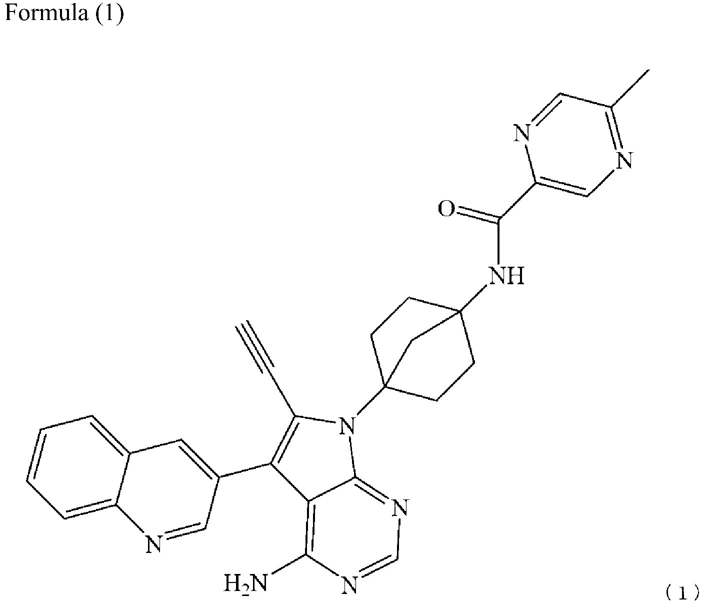

According to one embodiment of the present invention, there is provided a brain-penetrable antitumor agent comprising compound (1) or a salt thereof, which shows an EGFR inhibitory effect against mutant EGFR and shows a therapeutic efficacy against brain tumor.

According to another embodiment of the present invention, there is provided the use of compound (1) or a salt thereof for the manufacture of an antitumor agent showing brain penetration, EGFR inhibitory activity against mutant EGFR and/or an antitumor effect.

According to one embodiment of the present invention, there is provided compound (1) or a salt thereof for the treatment of a tumor, particularly brain tumor, which shows brain penetration, EGFR inhibitory activity against mutant EGFR and/or an antitumor effect.

According to yet another embodiment of the present invention, there is provided a therapeutic method for a tumor, particularly brain tumor, which comprises administering an antitumor agent comprising compound (1) or a salt thereof, which shows brain penetration and/or EGFR inhibitory activity against mutant EGFR.

According to one embodiment of the present invention, there is provided an anti-brain tumor agent comprising compound (1) or a salt thereof, which shows brain penetration and/or an EGFR inhibitory effect against mutant EGFR and shows a therapeutic efficacy against brain tumor.

According to another embodiment of the present invention, there is provided the use of compound (1) or a salt thereof for the manufacture of an anti-brain tumor agent showing brain penetration and/or EGFR inhibitory activity against mutant EGFR and/or an antitumor effect.

According to one embodiment of the present invention, there is provided compound (1) or a salt thereof for the treatment of a tumor, particularly brain tumor, with an anti-brain tumor agent showing brain penetration and/or mutant EGFR inhibitory activity and/or an anti-brain tumor effect.

According to yet another embodiment of the present invention, there is provided a therapeutic method for a tumor, particularly brain tumor, which comprises administering an anti-brain tumor agent comprising compound (1) or a salt thereof, which shows brain penetration and/or mutant EGFR inhibitory activity.

According to one embodiment of the present invention, there is provided a therapeutic agent for brain tumor comprising compound (1) or a salt thereof, which shows brain penetration and/or an EGFR inhibitory effect against mutant EGFR and shows a therapeutic efficacy against brain tumor.

According to one embodiment of the present invention, there is provided the use of compound (1) or a salt thereof for the manufacture of a therapeutic agent for brain tumor which shows brain penetration, mutant EGFR inhibitory activity and/or an antitumor effect.

According to one embodiment of the present invention, there is provided compound (1) or a salt thereof for the treatment of a tumor, particularly brain tumor, with a therapeutic agent for brain tumor which shows brain penetration, mutant EGFR inhibitory activity and/or an anti-brain tumor effect.

According to yet another embodiment of the present invention, there is provided a therapeutic method for a tumor, particularly brain tumor, which comprises administering a therapeutic agent for brain tumor comprising compound (1) or a salt thereof, which shows brain penetration, mutant EGFR inhibitory activity and/or an antitumor effect.

According to one embodiment of the present invention, there is provided a brain-penetrable EGFR inhibitor comprising compound (1) or a salt thereof, which shows an antitumor effect.

According to one embodiment of the present invention, there is provided the use of compound (1) or a salt thereof for the manufacture of a brain-penetrable EGFR inhibitor which shows mutant EGFR inhibitory activity and/or an antitumor effect.

According to one embodiment of the present invention, there is provided compound (1) or a salt thereof for the treatment of a tumor, particularly brain tumor, with a brain-penetrable EGFR inhibitor which shows mutant EGFR inhibitory activity and/or an anti-brain tumor effect.

According to yet another embodiment of the present invention, there is provided a therapeutic method for a tumor, particularly brain tumor, which comprises administering a brain-penetrable EGFR inhibitor comprising compound (1) or a salt thereof, which shows mutant EGFR inhibitory activity and/or an antitumor effect.

As used herein, a brain-penetrable antitumor agent is intended to mean an antitumor agent which causes at least a part of the active ingredient to penetrate into the brain and thereby exerts a therapeutic effect on primary brain tumor and/or metastatic brain tumor.

As used herein, an anti-brain tumor agent is intended to mean an antitumor agent which causes at least a part of the active ingredient to penetrate into the brain and thereby exerts a therapeutic effect on primary brain tumor and/or metastatic brain tumor.

As used herein, a therapeutic agent for brain tumor is intended to mean a therapeutic agent which causes at least a part of the active ingredient to penetrate into the brain and thereby exerts a therapeutic effect on primary brain tumor and/or metastatic brain tumor.

As used herein, a brain-penetrable EGFR inhibitor is intended to mean an EGFR inhibitor which causes at least a part of the active ingredient to penetrate into the brain and thereby exerts a therapeutic effect on primary brain tumor and/or metastatic brain tumor.

As found herein, the compound represented by formula (1) is also simply expressed as compound (1).

Compound (1) and a salt thereof may be prepared by known organic synthesis procedures. For example, they may be prepared according to the procedures described in Patent Document 1 or elsewhere.

If compound (1) has optical isomers, stereoisomers, rotational isomers, tautomers and other isomers, all of these isomers and mixtures thereof also fall within compound (1), unless otherwise specified. For example, if compound (1) has optical isomers, the racemic mixture and optical isomers resolved therefrom also fall within compound (1), unless otherwise specified.

A salt of compound (1) is intended to mean a pharmaceutically acceptable salt, as exemplified by a base addition salt or an acid addition salt.

Specific examples of such a salt include acid addition salts with inorganic acids such as hydrochloric acid, hydrobromic acid, hydroiodic acid, sulfuric acid, nitric acid, phosphoric acid and so on; acid addition salts with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, paratoluenesulfonic acid, glutamic acid, sorbic acid and so on; base addition salts with inorganic bases such as sodium, potassium, magnesium, calcium, aluminum and so on; base addition salts with organic bases such as methylamine, ethylamine, meglumine, ethanolamine and so on; or salts with basic amino acids such as lysine, arginine, ornithine and so on; and ammonium salt.

The term "pharmaceutically acceptable salt" is intended to mean a salt which has the compound's desired pharmacological activity and which is prepared from a pharmaceutically acceptable non-toxic base or acid, including an inorganic or organic base and an inorganic or organic acid.

Compound (1) or a salt thereof also includes a prodrug thereof. Such a prodrug refers to a compound which is converted into compound (1) or a salt thereof under physiological conditions *in vivo* through reaction with an enzyme or gastric acid, etc., i.e., a compound which is converted into compound (1) or a salt thereof by enzymatically causing oxidation, reduction, hydrolysis, etc. Moreover, such a prodrug may be a compound which is converted into compound (1) or a salt thereof under physiological conditions as described in "Pharmaceutical Research and Development," Vol. 7 (Molecule Design), pages 163 to 198, published in 1990 by Hirokawa Publishing Co., Japan.

Compound (1) or a salt thereof may be in either amorphous or crystalline form, and the crystalline form may be a single crystalline form or a polymorphic mixture, both of which fall within compound (1) or a salt thereof. Such crystalline forms may be prepared by being crystallized with the application of known crystallization techniques. Compound (1) or a salt thereof may be either a solvate (e.g., a hydrate) or a non-solvate, both of which fall within compound (1) or a salt thereof. Compound (1) or a salt thereof also encompasses those labeled with isotopes (e.g., ³H, ¹⁴C, ³⁵S, ¹²⁵I), etc. Although a plurality of crystals (crystalline polymorphs) differing in their spatially regular atomic arrangement and physicochemical properties may be generated, the salt of the compound to be used in the present invention may be any of these crystalline polymorphs, or may be a mixture of two or more crystalline polymorphs, or even a mixture of crystalline and amorphous forms.

As used herein, the term "effective amount" of compound (1) refers to the amount (therapeutically effective amount) of compound (1), which is required to cause biological or medical responses (e.g., reduction or inhibition of enzyme and/or protein activity) in a subject or which is required to ameliorate symptoms, alleviate conditions, slow or delay the progress of a disease, or prevent a disease, etc.

As used herein, the term "treatment" or "treating" encompasses post-operative adjuvant chemotherapy which is conducted after surgical excision of tumor to avoid recurrence, and pre-operative neoadjuvant chemotherapy which is conducted in advance for surgical excision of tumor.

As used herein, the term "subject" encompasses mammals and non-mammals. In one embodiment, the subject is a human, and may be a human who has been diagnosed as being in need of treatment for the symptoms, conditions or diseases disclosed herein.

There is no particular limitation on the age of the subject to be administered with a brain-penetrable antitumor agent, an anti-brain tumor agent, a therapeutic agent for brain tumor and/or a brain-penetrable EGFR inhibitor in one embodiment of the present invention. A brain-penetrable antitumor agent, an anti-brain tumor agent, a therapeutic agent for brain tumor and/or a brain-penetrable EGFR inhibitor in one embodiment of the present invention may be used not only in adults, but also in elderly subjects or children.

Compound (1) or a salt thereof or a brain-penetrable antitumor agent, an anti-brain tumor agent, a therapeutic agent for brain tumor and/or a brain-penetrable EGFR inhibitor, each comprising compound (1) or a salt thereof, in one embodiment of the present invention has excellent EGFR inhibitory activity. Moreover, they have excellent selectivity for EGFR. Thus, compound (1) or a salt thereof or a brain-penetrable antitumor agent, an anti-brain tumor agent, a therapeutic agent for brain tumor and/or a brain-penetrable EGFR inhibitor, each comprising compound (1) or a salt thereof, in one embodiment of the present invention is useful as an antitumor agent for malignant tumors with EGFR overexpression, EGFR gene amplification, EGFR mutation, etc.

As used herein, the term "wild-type EGFR" is intended to mean EGFR represented, for example, by the amino acid sequence (SEQ ID NO: 1) of GenBank Accession No. NP_005219.2. As used herein, the term "exon 18" refers to a region at positions 688 to 728 in the amino acid sequence of wild-type EGFR (e.g., GenBank Accession No. NP_005219.2).

As used herein, the term "exon 19" refers to a region at positions 729 to 760 in the amino acid sequence of wild-type EGFR (e.g., GenBank Accession No. NP_005219.2).

As used herein, the term "exon 20" refers to a region at positions 761 to 823 in the amino acid sequence of wild-type EGFR (e.g., GenBank Accession No. NP_005219.2).

As used herein, the term "exon 21" refers to a region at positions 824 to 875 in the amino acid sequence of wild-type EGFR (e.g., GenBank Accession No. NP_005219.2).

As used herein, the term "Ex19del" refers to a mutation with a deletion of one or more amino acids in the exon 19 region of wild-type EGFR. In addition to a deletion in this region, this mutation may comprise an insertion of one or more any amino acids. Examples of such an exon 19 deletion mutation include a mutation with a deletion of 5 amino acids covering from glutamic acid at position 746 to alanine at position 750 in the exon 19 region (Del E746-A750 (or also referred to as d746-750)), a mutation with a deletion of 7 amino acids covering from leucine at position 747 to proline at position 753 in the exon 19 region and an insertion of serine (Del L747-P753insS), a mutation with a deletion of 5 amino acids covering from leucine at position 747 to threonine at position 751 in the exon 19 region (Del L747-T751), and a mutation with a deletion of 4 amino acids covering from leucine at position 747 to alanine at position 750 in the exon 19 region and an insertion of proline (Del L747-A750insP), etc. Preferred may be a mutation with a deletion of 5 amino acids covering from glutamic acid at position 746 to alanine at position 750 in the exon 19 region (Del E746-A750), but is not limited thereto.

As used herein, the term "L718X" refers to a point mutation with a substitution of any amino acid for leucine encoded by codon 718 in exon 18 of wild-type EGFR. Examples of an amino acid substituted for leucine may include glutamine (L718Q), valine (L718V) and so on, but are not limited thereto.

As used herein, the term "G724X" refers to a point mutation with a substitution of any amino acid for glycine encoded by codon 724 in exon 18 of wild-type EGFR. Examples of an amino acid substituted for glycine may include serine (G724S) and so on, but are not limited thereto.

As used herein, the term "T790X" refers to a point mutation with a substitution of any amino acid for threonine encoded by codon 790 in exon 20 of wild-type EGFR. Examples of an amino acid substituted for threonine may include methionine (T790M) and so on, but are not limited thereto. T790X encompasses any amino acid mutations as long as they are *de novo* mutations or mutations caused upon use of first- or second-generation EGFR inhibitors. Such first- and second-generation EGFR inhibitors may be exemplified by gefitinib, erlotinib, afatinib, etc., but are not limited thereto.

As used herein, the term "L792X" refers to a point mutation with a substitution of any amino acid for leucine encoded by codon 792 in exon 20 of wild-type EGFR. Examples of an amino acid substituted for leucine may include phenylalanine (L792F), histidine (L792H), tyrosine (L792Y), valine (L792V), proline (L792P) and so on, but are not limited thereto.

As used herein, the term "G796X" refers to a point mutation with a substitution of any amino acid for glycine encoded by codon 796 in exon 20 of wild-type EGFR. Examples of an amino acid substituted for glycine may include arginine (G796R), serine (G796S), cysteine (G796C) and so on, but are not limited thereto.

As used herein, the term "C797X" refers to a point mutation with a substitution of any amino acid for cysteine encoded by codon 797 in exon 20 of wild-type EGFR. Examples of an amino acid substituted for cysteine may include serine (C797S), glycine (C797G) and so on, but are not limited thereto. C797X encompasses any amino acid mutations as long as they are mutations caused upon use of EGFR inhibitors covalently bonding to C797 (e.g., second-generation EGFR inhibitors and/or third-generation EGFR inhibitors). Such second-generation EGFR inhibitors may be exemplified by afatinib, etc., but are not limited thereto. Likewise, such third-generation EGFR inhibitors may be exemplified by osimertinib, lazertinib, etc., but are not limited thereto.

As used herein, the term "T854X" refers to a point mutation with a substitution of any amino acid for threonine at codon 854 in exon 21 of wild-type EGFR. Examples of an amino acid substituted for threonine may include alanine (T854A), isoleucine (T854I) and so on, but are not limited thereto.

As used herein, the term "L858X" refers to a point mutation with a substitution of any amino acid for leucine at codon 858 in exon 21 of wild-type EGFR. Examples of an amino acid substituted for leucine may include arginine (L858R), methionine (L858M) and so on, but are not limited thereto.

As used herein, the term "mutant EGFR" refers to EGFR discussed herein. In one embodiment of the present invention, compound (1) has an inhibitory effect against "Ex19del," "L718X," "G724X," "T790X," "L792X," "G796X," "C797X," "T854X" and "L858X" (where X represents any amino acid) and has a therapeutic effect on a tumor having one or more of these mutant EGFRs, but the type of mutation is not limited thereto.

In one embodiment of the present invention, mutant EGFR which may be inhibited by compound (1) is Ex19del. Among candidates for Ex19del, preferred is at least one selected from Del E746-A750, Del L747-P753insS, Del L747-T751 and Del L747-A750insP, but is not limited thereto.

In one embodiment of the present invention, mutant EGFR which may be inhibited by compound (1) is C797X (where X represents any amino acid). Among candidates for C797X, preferred is at least one selected from C797S and C797G, and particularly preferred is C797S, but is not limited thereto.

In one embodiment of the present invention, mutant EGFR which may be inhibited by compound (1) is L718X (where X represents any amino acid). Among candidates for L718X, preferred is at least one selected from L718Q and L718V, but is not limited thereto.

In one embodiment of the present invention, mutant EGFR which may be inhibited by compound (1) is G724X (where X represents any amino acid), and preferred is G724S, but is not limited thereto.

In one embodiment of the present invention, mutant EGFR which may be inhibited by compound (1) is L792X (where X represents any amino acid), and preferred is at least one selected from L792F, L792H, L792Y, L792V and L792P, but is not limited thereto.

In one embodiment of the present invention, mutant EGFR which may be inhibited by compound (1) is G796X (where X represents any amino acid), and preferred is at least one selected from G796R, G796S and G796C, but is not limited thereto.

In one embodiment of the present invention, mutant EGFR which may be inhibited by compound (1) is T854X (where X represents any amino acid), and preferred is at least one selected from T854A and T854I, but is not limited thereto.

In one embodiment of the present invention, mutant EGFR which may be inhibited by compound (1) may have at least one mutation selected from Ex19del, T790M and L858X (where X represents any amino acid) in addition to the above mutations. Among candidates for L858X, preferred is at least one selected from L858R and L858M, and particularly preferred is L858R, but is not limited thereto.

In one embodiment of the present invention, mutant EGFR which may be inhibited by compound (1) preferably has not only C797S, but also one or more mutations selected from EGFR mutations consisting of Ex19del, L858R and T790M.

As used herein, the term "point mutation" refers to a mutation which provides a substitution, insertion or deletion of one or more (e.g., 1 to around 10, preferably 1 to around 5, more preferably 1, 2 or around 3) amino acid residues, and may include a nucleic acid in-frame insertion and/or deletion mutation.

Moreover, compound (1) or a salt thereof or a brain-penetrable antitumor agent, an anti-brain tumor agent, a therapeutic agent for brain tumor and/or a brain-penetrable EGFR inhibitor, each comprising compound (1) or a salt thereof, in one embodiment of the present invention has excellent selectivity for mutant EGFR, and has the advantage of causing fewer side effects from wild-type EGFR or other kinases.

Further, compound (1) or a salt thereof or a brain-penetrable antitumor agent, an anti-brain tumor agent, a therapeutic agent for brain tumor and/or a brain-penetrable EGFR inhibitor, each comprising compound (1) or a salt thereof, in one embodiment of the present invention has the advantage of being available for use in subjects with a mutation(s) occurring upon use of a third-generation EGFR inhibitor (e.g., subjects who develop osimertinib resistance after treatment with osimertinib or subjects who develop lazertinib resistance after treatment with lazertinib).

As used herein, the term "osimertinib resistance" is intended to mean a state where the therapeutic effect of osimertinib disappears or is attenuated in subjects receiving osimertinib to treat EGFR gene mutation-positive tumors (peripheral cancers (primary lesions), primary brain tumor and/or metastatic brain tumor). Likewise, the term "lazertinib resistance" is intended to mean a state where the therapeutic effect of lazertinib disappears or is attenuated in subjects receiving lazertinib to treat EGFR gene mutation-positive tumors (peripheral cancers (primary lesions), primary brain tumor and/or metastatic brain tumor).

As used herein, the phrase "after treatment with osimertinib" is intended to mean being administered after osimertinib administration. Likewise, the phrase "after treatment with lazertinib" is intended to mean being administered after lazertinib administration.

Compound (1) or a salt thereof or a brain-penetrable antitumor agent, an anti-brain tumor agent, a therapeutic agent for brain tumor and/or a brain-penetrable EGFR inhibitor, each comprising compound (1) or a salt thereof, in one embodiment of the present invention is useful as a medicament for preventing or treating EGFR-related diseases because of its excellent EGFR inhibitory activity.

One embodiment of the present invention provides an EGFR inhibitor comprising compound (1) or a salt thereof. One embodiment of the present invention provides an EGFR inhibitor comprising compound (1) or a salt thereof for use after treatment with osimertinib. Alternatively, one embodiment of the present invention provides an EGFR inhibitor comprising compound (1) or a salt thereof for use after treatment with lazertinib.

Another embodiment of the present invention provides an antitumor agent, an anti-brain tumor agent and/or a therapeutic agent for brain tumor, each comprising compound (1) or a salt thereof, for use after treatment with osimertinib. Alternatively, another embodiment of the present invention provides an antitumor agent, an anti-brain tumor agent and/or a therapeutic agent for brain tumor, each comprising compound (1) or a salt thereof, for use after treatment with lazertinib.

In addition, one embodiment of the present invention provides a therapeutic method for a tumor, particularly brain tumor, which comprises administering an effective amount of compound (1) or a salt thereof to a subject in need thereof after treatment with osimertinib. Further, one embodiment of the present invention provides a therapeutic method for a tumor, particularly brain tumor, which comprises administering an effective amount of compound (1) or a salt thereof to a subject in need thereof after treatment with lazertinib.

One embodiment of the present invention provides the use of compound (1) or a salt thereof for the manufacture of an antitumor agent, an anti-brain tumor agent and/or a therapeutic agent for brain tumor for use after treatment with osimertinib. Further, one embodiment of the present invention provides the use of compound (1) or a salt thereof for the manufacture of an antitumor agent, an anti-brain tumor agent and/or a therapeutic agent for brain tumor for use after treatment with lazertinib.

One embodiment of the present invention provides compound (1) or a salt thereof for use in the treatment of a tumor, particularly brain tumor, after treatment with osimertinib. Further, one embodiment of the present invention provides compound (1) or a salt thereof for use in the treatment of a tumor, particularly brain tumor, after treatment with lazertinib.

Compound (1) or a salt thereof is useful as a medicament for preventing or treating mutant EGFR-related diseases because of its excellent mutant EGFR inhibitory activity.

The term "mutant EGFR-related disease" refers to a disease whose incidence rate is reduced upon elimination, suppression and/or inhibition of the EGFR function or whose symptoms are remitted, alleviated and/or completely cured upon elimination, suppression and/or inhibition of the EGFR function. Examples of such a disease include malignant tumors, etc., but are not limited thereto. Malignant tumors are preferably those with enhanced activation of EGFR, more preferably head and neck cancer, thyroid cancer, salivary gland cancer, esophageal cancer, gastric cancer (digestive system cancer), duodenal cancer, liver cancer, biliary tract cancer (e.g., gallbladder and bile duct cancer), pancreatic cancer, colorectal cancer (e.g., colon cancer, rectal cancer), lung cancer (e.g., non-small cell lung cancer, small cell lung cancer, mesothelioma), breast cancer, ovarian cancer, uterine cancer (e.g., uterine cervical cancer, uterine body cancer), renal cancer, renal pelvis and ureter cancer, bladder cancer, prostate cancer, testicular tumor, leukemia, malignant lymphoma, multiple myeloma, bone and soft tumor, skin cancer, malignant melanoma, adrenal gland tumor, brain tumor and other tumors with enhanced activation of EGFR. Preferred are salivary gland cancer, lung cancer (e.g., non-small cell lung cancer, small cell lung cancer, mesothelioma), colorectal cancer (e.g., colon cancer, rectal cancer), thyroid cancer, breast cancer, leukemia, skin cancer, malignant melanoma and brain tumor. Preferred are salivary gland cancer, lung cancer (e.g., non-small cell lung cancer, small cell lung cancer, mesothelioma), colorectal cancer (e.g., colon cancer, rectal cancer), thyroid cancer, breast cancer, pancreatic cancer, leukemia, skin cancer, malignant melanoma and brain tumor, and more preferred are salivary gland cancer, lung cancer, breast cancer, pancreatic cancer, colorectal cancer, ovarian cancer, thyroid cancer, skin cancer, malignant melanoma and brain tumor. More particularly preferred are non-small cell lung cancer, breast cancer, colorectal cancer, thyroid cancer and brain tumor.

An enhanced activation of EGFR is intended to mean that EGFR activation is enhanced, e.g., when the EGFR gene is translocated, mutated (including point mutation, deletion mutation and insertion mutation) or overexpressed (including a state where the copy number of the EGFR gene is increased, where messenger RNA of EGFR is expressed in excess, where the EGFR protein is increased, or where the EGFR protein is constitutively activated).

Target cancers or tumors are not limited in any way, but examples include epithelial cancer (e.g., respiratory system cancer, digestive system cancer, reproductive system cancer, secretory system cancer), sarcoma, hematopoietic cell tumor, central nervous system tumor, peripheral nerve tumor, etc.

Specific cancer types include head and neck cancer, thyroid cancer, salivary gland cancer, esophageal cancer, gastric cancer (digestive system cancer), duodenal cancer, liver cancer, biliary tract cancer (e.g., gallbladder and bile duct cancer), pancreatic cancer, colorectal cancer (e.g., colon cancer, rectal cancer), lung cancer (e.g., non-small cell lung cancer, small cell lung cancer, mesothelioma), breast cancer, ovarian cancer, uterine cancer (e.g., uterine cervical cancer, uterine body cancer), renal cancer, renal pelvis and ureter cancer, bladder cancer, prostate cancer, testicular tumor, leukemia, malignant lymphoma, multiple myeloma, bone and soft tumor, skin cancer, malignant melanoma, adrenal gland tumor, brain tumor, etc. Preferred are salivary gland cancer, lung cancer (e.g., non-small cell lung cancer, small cell lung cancer, mesothelioma), colorectal cancer (e.g., colon cancer, rectal cancer), thyroid cancer, breast cancer, leukemia, skin cancer, malignant melanoma and brain tumor. Preferred are salivary gland cancer, lung cancer (e.g., non-small cell lung cancer, small cell lung cancer, mesothelioma), colorectal cancer (e.g., colon cancer, rectal cancer), thyroid cancer, breast cancer, pancreatic cancer, leukemia, skin cancer, malignant melanoma and brain tumor, and more preferred are salivary gland cancer, lung cancer, breast cancer, pancreatic cancer, colorectal cancer, ovarian cancer, thyroid cancer, skin cancer, malignant melanoma and brain tumor. More particularly preferred are non-small cell lung cancer, breast cancer, colorectal cancer, thyroid cancer and brain tumor.

In a typical embodiment of the present invention, the target tumor for which compound (1) exerts its effect by passing through the BBB is brain tumor. As mentioned herein, brain tumor includes primary brain tumor and metastatic brain tumor. Examples of primary tumor include, but are not limited to, glioma, primary malignant lymphoma of the central nervous system, meningioma, pituitary adenoma, neurilemmoma, craniopharyngioma, etc. Metastatic brain tumor has no limitation on the type of its primary tumor.

In the case of metastatic brain tumor, the type of its primary tumor is not limited in any way, but specific cancer types include head and neck cancer, digestive organ cancer (e.g., esophageal cancer, gastric cancer, duodenal cancer, liver cancer, biliary tract cancer (e.g., gallbladder and bile duct cancer), pancreatic cancer, large bowel cancer (e.g., colorectal cancer, colon cancer, rectal cancer, anal cancer)), lung cancer (e.g., non-small cell lung cancer, small cell lung cancer, mesothelioma (e.g., pleural mesothelioma, peritoneal mesothelioma, pericardial mesothelioma, testicular mesothelioma)), breast cancer, genital cancer (e.g., ovarian cancer, vulvar cancer, uterine cancer (e.g., uterine cervical cancer, uterine body cancer, endometrial cancer)), urinary organ cancer (e.g., renal cancer, bladder cancer, prostate cancer, testicular tumor, urothelial cancer, renal pelvis cancer, urethral cancer), hematopoietic tumor (e.g., leukemia, malignant lymphoma, multiple myeloma), bone and soft tumor, rhabdomyosarcoma, skin cancer, malignant neurilemmoma, neuroendocrine tumor, thyroid cancer, etc. Preferred are head and neck cancer, breast cancer, large bowel cancer, esophageal cancer, pancreatic cancer, lung cancer, ovarian cancer, renal cancer, bladder cancer and skin cancer, and particularly preferred is lung cancer.

Even if a drug has passed the BBB, there may be a case where the drug is actively discharged to the bloodstream side by transporters such as P-glycoprotein (hereinafter referred to as P-gp) or breast cancer resistance protein (hereinafter referred to as BCRP) localized on the blood vessel side of brain capillary endothelial cells, making it difficult to maintain a concentration that exerts therapeutic effects. In order to exhibit therapeutic effects against primary and/or metastatic brain tumors, it is preferable to avoid becoming a substrate for these transporters. Whether or not a drug becomes a substrate for these transporters can be confirmed by a transcellular transport test using cells expressing transporters such as P-gp or BCRP, as in Test Example 7 in the Examples. According to Non-patent Literature 14, if the efflux ratio (herein, it is synonymous with the "ratio of apparent permeability coefficients (Papp)") is less than 2, it can be determined that the drug is not a substrate for these transporters.

The penetration of compound (1) into the brain may be evaluated using the brain-to-plasma drug concentration ratio (Kp value) and/or the brain-to-plasma unbound drug concentration ratio (Kp,uu value). In the evaluation of mouse brain penetration, if the Kp value is 0.1 or higher when the cut-off value is set to 0.1, the compound is regarded as having brain penetration. In the present invention, a preferred Kp value is 0.1 or higher. Likewise, the Kp,uu value correlates with penetration into the brain, and if the Kp,uu value is 0.3 or higher, brain penetration can be determined to be good. A higher Kp,uu value indicates higher brain penetration and is therefore preferred. The Kp,uu value is preferably 0.4 or higher, more preferably 0.5 or higher.

Because of having an inhibitory effect against mutant EGFR, compound (1) inhibits mutant EGFR in a tumor (primary or metastatic) occurring in brain tissue, and can be used to suppress tumor growth. Moreover, due to its high selectivity, compound (1) is particularly useful in the treatment of brain tumor (primary or metastatic) in which an EGFR mutation is involved in cancer cell proliferation.

Compound (1) or a salt thereof or a brain-penetrable antitumor agent, an anti-brain tumor agent, a therapeutic agent for brain tumor and/or a brain-penetrable EGFR inhibitor, each comprising compound (1) or a salt thereof, in one embodiment of the present invention can also be used to prevent the development of brain tumor. Compound (1) is able to pass through the BBB, and therefore may be used to prevent cancer cells having an EGFR mutation from metastasis and colonization in the brain. Namely, in one embodiment of the present invention, compound (1) may also be used to prevent the development of metastatic brain tumor. Further, in one embodiment of the present invention, compound (1) shows excellent brain penetration, and therefore also allows the simultaneous treatment of peripheral cancer (primary lesion) having an EGFR mutation and brain tumor having an EGFR mutation.

A brain-penetrable antitumor agent, an anti-brain tumor agent, a therapeutic agent for brain tumor and/or a brain-penetrable EGFR inhibitor in one embodiment of the present invention may be provided in the form of its active ingredient alone. Alternatively, a brain-penetrable antitumor agent, an anti-brain tumor agent, a therapeutic agent for brain tumor and/or a brain-penetrable EGFR inhibitor in one embodiment of the present invention may optionally comprise a pharmaceutically acceptable carrier and so on, in addition to its active ingredient, i.e., compound (1) or a salt thereof. Thus, a brain-penetrable antitumor agent, an anti-brain tumor agent, a therapeutic agent for brain tumor and/or a brain-penetrable EGFR inhibitor in one embodiment of the present invention may be prepared as a pharmaceutical composition consisting of one ingredient or comprising two or more ingredients. One aspect of the present invention provides a pharmaceutical composition comprising compound (1) or a salt thereof. A pharmaceutical composition in one embodiment of the present invention comprises compound (1) or a salt thereof, and a pharmaceutically acceptable carrier. Alternatively, one embodiment of the present invention provides the use of compound (1) or a salt thereof for the manufacture of a brain-penetrable antitumor agent, an anti-brain tumor agent, a therapeutic agent for brain tumor and/or a brain-penetrable EGFR inhibitor or a pharmaceutical composition. Another embodiment of the present invention provides compound (1) or a salt thereof for use as a brain-penetrable antitumor agent, an anti-brain tumor agent, a therapeutic agent for brain tumor and/or a brain-penetrable EGFR inhibitor or a medicament.

A brain-penetrable antitumor agent, an anti-brain tumor agent, a therapeutic agent for brain tumor and/or a brain-penetrable EGFR inhibitor in one embodiment of the present invention may be prepared into various dosing formulations in a known manner, optionally by using a pharmaceutically acceptable carrier. The mode of administration may be either oral or parenteral. Such formulations may be of any form, as exemplified by oral formulations (e.g., tablets, coated tablets, pills, powders, granules, capsules, solutions, suspensions, emulsions) and parenteral formulations (e.g., injections, suppositories, inhalants).

Examples of a pharmaceutically acceptable carrier available for use include various organic or inorganic carrier substances conventionally used as formulation materials, as exemplified by excipients, binders, disintegrants, lubricants, coating agents and so on in solid formulations, or which may be incorporated as solvents, solubilizers, suspending agents, isotonizing agents, buffering agents, soothing agents or the like in liquid formulations. Moreover, formulation additives may also be used as needed, as exemplified by antiseptics, antioxidants, coloring agents, sweeteners, stabilizers, etc.

Examples of excipients include starches, sugars, polysaccharides, inorganic compounds and so on. Examples of starches include potato starch, corn starch, rice starch, partially pregelatinized starch and so on. Examples of sugars include monosaccharides, disaccharides, trisaccharides and sugar alcohols, as exemplified by lactose, sucrose, trehalose, D-mannitol, raffinose, xylitol, erythritol, etc. Examples of polysaccharides include cellulose, dextran and so on, as exemplified by crystalline cellulose, hydroxypropyl cellulose, hydroxypropyl methylcellulose, etc. Examples of inorganic compounds include silicic acids and so on, as exemplified by light anhydrous silicic acid, calcium silicate, etc.

Examples of binders include hydroxypropyl cellulose, methylcellulose, polyvinylpyrrolidone, polyvinyl alcohol, dried glucose syrup, hypromellose and so on.

Examples of disintegrants include sodium starch glycolate, carmellose calcium, croscarmellose sodium, crospovidone, low-substituted hydroxypropyl cellulose, partially pregelatinized starch and so on.

Examples of lubricants include talc, magnesium stearate, sucrose fatty acid esters, stearic acid, sodium stearyl fumarate and so on.

Examples of coating agents include ethylcellulose, aminoalkyl methacrylate copolymer RS, hypromellose, sucrose and so on.

Examples of solvents include water, propylene glycol, and physiological saline.

Examples of solubilizers include polyethylene glycol, alcohols (e.g., ethanol), cyclodextrin, cyclodextrin derivatives, ionic surfactants, nonionic surfactants and so on, as exemplified by sorbitan fatty acid esters (e.g., Polysorbate 80), sucrose fatty acid esters, sodium lauryl sulfate, etc.

Examples of suspending agents include carrageenan, crystalline cellulose-carmellose sodium, polyoxyethylene hydrogenated castor oil, gum arabic, and sodium alginate.

Examples of isotonizing agents include sodium chloride, glycerin, potassium chloride and so on.

Examples of pH adjusters or buffering agents include sodium citrate, hydrochloric acid, lactic acid, phosphoric acid, sodium dihydrogen phosphate and so on.

Examples of soothing agents include procaine hydrochloride, lidocaine and so on.

Examples of antiseptics include ethyl parahydroxybenzoate, cresol, benzalkonium chloride and so on.

Examples of antioxidants include sodium sulfite, ascorbic acid, natural vitamin E and so on.

Examples of coloring agents include titanium oxide, iron sesquioxide, Food Blue No. 1, copper chlorophyll and so on.

Examples of flavoring agents include aspartame, saccharin, sucralose, l-menthol, mint flavor and so on.

Examples of stabilizers include sodium pyrosulfite, sodium edetate, erythorbic acid, magnesium oxide, dibutylhydroxytoluene and so on.

In the case of preparing oral solid formulations, compound (1) may be mixed with an excipient and optionally with a binder, a disintegrant, a lubricant, a coloring agent, a flavoring agent, etc., and then formulated in a standard manner to prepare tablets, coated tablets, granules, powders, capsules, etc.

In the case of preparing injections, compound (1) may be mixed with a pH adjuster or a buffering agent, a stabilizer, an isotonizing agent, a local anesthetic agent, etc., and then formulated in a standard manner to prepare injections for subcutaneous, intramuscular and intravenous use.

The amount of compound (1) to be incorporated into the above dosage unit forms will vary depending on the symptom of a patient to be applied thereby or the dosage form thereof, etc. However, in general, it is desirable to incorporate compound (1) in an amount of about 0.05 to 1000 mg for oral formulations, in an amount of about 0.1 to 500 mg for injections, and in an amount of about 1 to 1000 mg for suppositories or external preparations, per dosage unit form.

Moreover, the daily dose of compound (1) in the above dosage forms will vary depending on the symptom, body weight, age and/or sex of a patient, etc., and cannot be determined simply. However, the daily dose of compound (1) for adults (body weight: 50 kg) may usually be set to about 0.05 to 5000 mg, preferably 0.1 to 1000 mg.

### EXAMPLES

The present invention will be further described in more detail by way of the following examples, but the present invention is not limited in any way by these examples. The present invention is fully described in the Example section, but those skilled in the art would understand that various changes and/or modifications will be possible. Thus, such changes and/or modifications fall within the present invention as long as they do not depart from the scope of the present invention.

In the following compound examples, % represents percent by weight, unless otherwise specified.

The various reagents used in the Example section were commercially available products, or those synthesized by the procedures described in the patent document or by known procedures, unless otherwise specified.

In addition, the meanings of abbreviations are shown below.
PBS: phosphate-buffered physiological saline
HPMC: hydroxypropyl methylcellulose
HP-β-CD: hydroxypropyl-beta-cyclodextrin

### Test Example 1 Evaluation of plasma protein binding and brain protein binding

Compound (1) was added to mouse plasma to give a final concentration of 5 µmol/L. The resulting compound-containing mouse plasma was added to the donor side of an equilibrium dialysis device (RED device system). PBS was added to the receiver side of the device, and the device was then incubated in a 5% CO₂ incubator for 8 hours, followed by LC-MS/MS to measure the concentrations of the compound on the donor and receiver sides. The ratio of the compound concentration on the receiver side to the compound concentration on the donor side was used to calculate the unbound rate of the compound to plasma proteins. The plasma protein unbound rate was shown to be 0.0008.

Compound (1) was added to a mouse brain homogenate, in place of mouse plasma, to give a final concentration of 5000 nmol/g, and the same procedure as shown above was repeated to calculate the unbound rate of the compound to mouse brain proteins, except that the dilution factor was taken into consideration for calculation of the unbound rate. The mouse brain homogenate used here was obtained by adding three volumes of 50 mmol/L sodium fluoride-containing PBS to the mouse brain. The brain protein unbound rate was shown to be 0.0017.

### Test Example 2 Evaluation of brain penetration in mice

To compound (1), a solution containing 20% HP-β-CD and 0.1 mol/L hydrochloric acid was added as a dosing solvent, and the resulting mixture was ground with an agate mortar to prepare a dosing formulation, which was then orally administered once to BALB/cAJcl-nu/nu mice (CLEA Japan, Inc., Japan) at a dose of 20, 40 or 80 mg/kg. At 0.25, 0.5, 1, 2, 4, 6, 8 and 24 hours after oral administration, blood was collected via the inferior vena cava under isoflurane anesthesia, and the brain was excised from each mouse after euthanasia by exsanguination. The resulting blood samples were centrifuged to obtain plasma samples. The resulting brain samples were diluted with three volumes of PBS and then homogenized with a bead impact homogenizer to thereby obtain brain homogenates.

The concentrations of the compound in the resulting plasma samples and brain homogenates were measured by LC-MS/MS. The compound concentration in brain was calculated by using a calibration curve prepared in consideration of the dilution factor of the brain homogenates, and the compound concentrations in plasma and brain were used to calculate AUC₀₋₂₄ in plasma and brain. The Kp value was calculated from the brain/plasma ratio of AUC₀₋₂₄. Moreover, the plasma protein unbound rate and the brain protein unbound rate determined in Test Example 1 were used to calculate unbound AUC₀₋₂₄ in plasma and brain, and the Kp,uu value was calculated from the brain/plasma ratio of unbound AUC₀₋₂₄. The results obtained are shown in Table 1. The Kp value was shown to be 0.319 to 0.691, while the Kp,uu value was shown to be 0.68 to 1.47. This result indicated that compound (1) showed good penetration into the brain.

**Table 1**

| Dose (mg/kg) | Compound (1) | | | |
|---|---|---|---|---|
| | AUC₀₋₂₄ | | AUC ratio | |
| | Plasma (ng·hr/mL) | Brain (ng·hr/g) | Kp | Kp,uu |
| 20 | 37300 | 11900 | 0.319 | 0.68 |
| 40 | 104000 | 52800 | 0.508 | 1.08 |
| 80 | 194000 | 134000 | 0.691 | 1.47 |
| Protein unbound rate | 0.0008 | 0.0017 | - | - |

### Test Example 3

Subsequently, comparative compound 1 having the structure of formula (2) shown below was used to calculate the Kp value at 0.5 hours after administration. It should be noted that comparative compound 1 was prepared according to the procedures described in Patent Document 1. The test was conducted under the same conditions as used in Test Example 2, except that after comparative compound 1 was orally administered once at a dose of 40 mg/kg, the brain was excised from each mice at 0.5 hours, and the resulting brain samples were diluted with three volumes of water and then homogenized with a ultrasonic homogenizer. The results obtained are shown in Table 2. The Kp value at 0.5 hours after administration was 0.832 in compound (1), but 0.0214 in comparative compound 1.

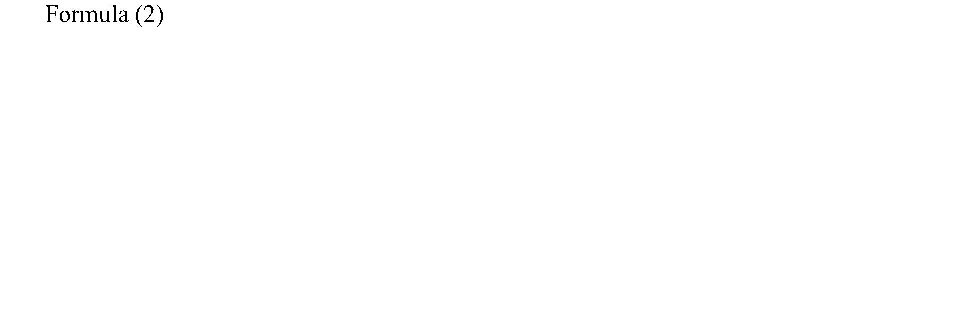

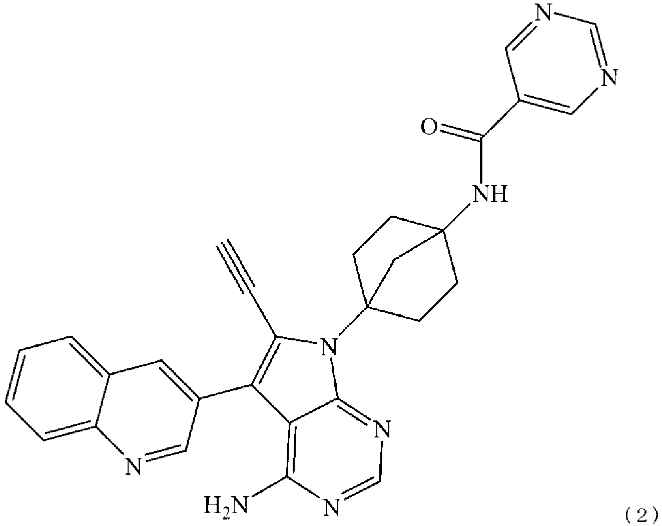

**Table 2**

| Compound | 0.5 hours after oral administration at a dose of 40 mg/kg | | |
|---|---|---|---|
| | Concentration | | Kp |
| | Plasma (ng/mL) | Brain (ng/g) | |
| Compound (1) | 12900 | 10800 | 0.832 |
| Comparative compound 1 | 6340 | 137 | 0.0214 |

### Test Example 4 Efficacy evaluation test of compound (1) and brigatinib in a mouse model with implantation into the brain

In a BALB/cAJcl-nu/nu mouse model transplanted intracranially with NIH/3T3 cells expressing EGFR with Ex19del (Del E746-A750), T790M and C797S mutations (ex19del/T790M/C797S) (SEQ ID NO: 2) and luciferase, compound (1) and brigatinib were verified for their effects on the growth of intracranial tumor and the survival time of the mice. Brigatinib has been used clinically in anaplastic lymphoma kinase (ALK) mutation-positive lung cancer, while brigatinib has been known to also inhibit EGFR with ex19del/T790M/C797S.

The cells were adjusted to 5.0 × 10⁶ cells/mL with PBS to prepare a cell suspension, which was stored on ice until transplantation. Male nude mice at 6 weeks of age (BALB/cAJcl-nu/nu, CLEA Japan, Inc., Japan) were each head-fixed under anesthesia with a stereotaxic apparatus, and 2 µL of the cell suspension was injected at 0.5 mm anterior, 2.0 mm right and 3.5 mm deep from the bregma, whereby 1.0 × 10⁴ cells per mouse were transplanted. The day of cell transplantation was defined as Day 0.

On Day 8, luciferin was intraperitoneally administered to measure the level of luminescence (Photon/sec) using an IVIS Lumina II Imaging System (PerkinElmer). According to the measured luminescence levels, the mice were divided into total 3 groups of 10 mice each such that the level of luminescence in each group was equal.

Compound (1) was orally administered every day at a dose of 80 mg/kg/day from Day 8 until Day 56 or until a humane endpoint was applied or the mice were dead. During the period from Day 50 until Day 56, in the compound (1)-receiving group, the dose was decreased to 50 mg/kg or the drug was withdrawn in mice having loose stools or diarrhea. Into a dosing solvent prepared to contain 0.5 w/v% HPMC and 0.1 mol/L hydrochloric acid, compound (1) was dissolved at 8.0 mg/mL to prepare a dosing solution. Brigatinib was orally administered every day at a dose of 50 mg/kg from Day 8 until Day 37 or until a humane endpoint was applied or the mice were dead. Brigatinib was dissolved into a dosing solvent prepared to contain 0.5 w/v% HPMC and 0.1 mol/L hydrochloric acid, thereby preparing a 5.0 mg/mL dosing solution. For use as a control group, a group receiving the dosing solvent was provided and administered in the same manner. Based on the results of body weight measurement for each mouse on the day of administration, the dosing solutions thus prepared were each administered at a dose of 10 mL/kg per mouse.

For evaluation of intracranial tumor growth, the level of luminescence was measured as described above on Day 8, Day 14 and Day 21. Moreover, the period until a humane endpoint was applied to each mouse or until the mouse was dead was defined as a survival time, and the median survival time was calculated therefrom. These animal tests were all conducted in accordance with the animal experiment guidelines of Taiho Pharmaceutical Co. Ltd., Japan (which went into effect in April 2013).

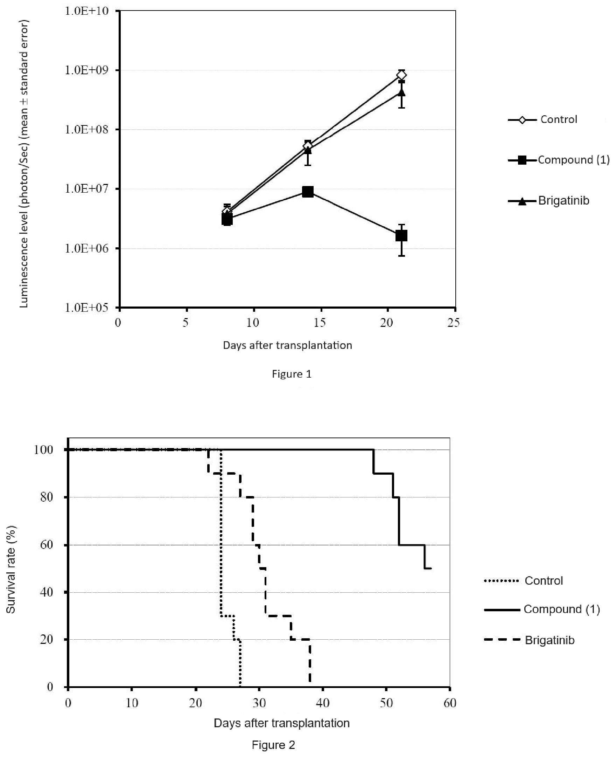

The results obtained are shown in Figure 1. With regard to the level of luminescence serving as an indicator for intracranial tumor growth, the control group showed a time-induced increase in the level of luminescence from Day 8 until Day 21, and the level of luminescence on Day 21 was 8.353 × 10⁸ ± 1.608 × 10⁸ p/s (photon/sec). In the brigatinib-receiving group, the level of luminescence on Day 21 was 4.303 × 10⁸ ± 1.970 × 10⁸ p/s, which was similar to that of the control group. On the other hand, in the compound (1)-receiving group, the level of luminescence on Day 21 was 1.648 × 10⁶ ± 8.929 × 10⁵ p/s. The compound (1)-receiving group was found to have a statistically significant difference relative to the control group (Dunnett type multiple comparison test, p<0.001), whereas the brigatinib-receiving group had no statistically significant difference. The statistical analysis was conducted by EXSUS (ver 10.0) via SAS (ver 9.4).

The results obtained are shown in Figure 2. With regard to the survival time of each group, the compound (1)-receiving group showed a statistically significantly longer survival time than the control group and the brigatinib-receiving group (p<0.001, log-rank test). Moreover, the brigatinib-receiving group also showed a statistically significant extension in the survival time when compared to the control group (p<0.001, log-rank test). The median survival time was 24.0 days in the control group, whereas the median survival time is 56.5 days in the compound (1)-receiving group and 30.5 days in the brigatinib-receiving group. The statistical analysis was conducted as described above.

The foregoing results indicated that in the mouse model transplanted intracranially with NIH/3T3 cells expressing EGFR with ex19del/T790M/C797S mutations and luciferase, compound (1) suppressed intracranial tumor growth and showed a greater extension in the survival time than brigatinib.

### Test Example 5 Efficacy evaluation test of compound (1) in mouse subcutaneous transplantation model

In a BALB/cAJcl-nu/nu mouse model transplanted subcutaneously with NIH/3T3 cells expressing EGFR with Ex19del (Del E746-A750), T790M and C797S mutations (ex19del/T790M/C797S) (SEQ ID NO: 2), compound (1) was verified for its effect on tumor growth, together with erlotinib and osimertinib.

On the day of cell transplantation, the cells were suspended at 2.0 × 10⁷ cells/mL in PBS and then mixed with an equal volume of Matrigel (Corning Inc.) to prepare a cell suspension of 1.0 × 10⁷ cells/mL, which was stored on ice until transplantation. After the mice were each immobilized, 0.1 mL per mouse of the cell suspension was injected into the right side of the chest using a 1-mL syringe equipped with a 25-G needle, whereby 1.0 × 10⁶ cells per mouse were transplanted.

In mice showing tumor engraftment, the long- and short-axis diameters of tumor were measured with an electronic caliper, and the tumor volume was determined according to the following equation. $Tumor volume \left({mm}^{3}\right) = long - axis diameter \left(mm\right) \times short - axis diameter {\left(mm\right)}^{2} / 2$

Mice whose tumor volume was 91.7914500 mm³ to 135.3264660 mm³ were selected and randomly divided into groups of 5 mice each. After grouping, the logarithmically transformed mean tumor volume of each group was confirmed to be not statistically significant by Turkey test. The statistical analysis was conducted by EXSUS (ver. 10.0.3) via SAS (ver. 9.4). The day of grouping was defined as Day 1.

Compound (1), erlotinib and osimertinib were orally administered every day from Day 1 until Day 10. Compound (1) was dissolved into a dosing solvent prepared to contain 20 w/v% HP-β-CD and 0.1 mol/L hydrochloric acid, thereby preparing 1.0 mg/mL, 2.0 mg/mL, 4.0 mg/mL and 8.0 mg/mL dosing solutions. Erlotinib was suspended in a dosing solvent prepared to contain 0.5 w/v% HPMC and 0.1 w/v% Tween 80, thereby preparing a 8.0 mg/mL dosing solution. Osimertinib was dissolved into a dosing solvent prepared to contain 0.5 w/v% HPMC and 0.1 mol/L hydrochloric acid, thereby preparing a 2.5 mg/mL dosing solution. Based on the results of body weight measurement for each mouse on the day of administration, the dosing solutions thus prepared were each administered at a dose of 10 mL/kg per mouse.

To evaluate the antitumor effects of compound (1), erlotinib and osimertinib, the tumor volumes on Days 1, 4, 8 and 11 were calculated for each mouse as described above to determine the mean tumor volume of each group. For comparison, a control group was provided to receive the dosing solvent for compound (1), and the tumor volume was determined in the same manner. These animal tests were all conducted in accordance with the animal experiment guidelines of Taiho Pharmaceutical Co. Ltd., Japan (which went into effect in April 2013).

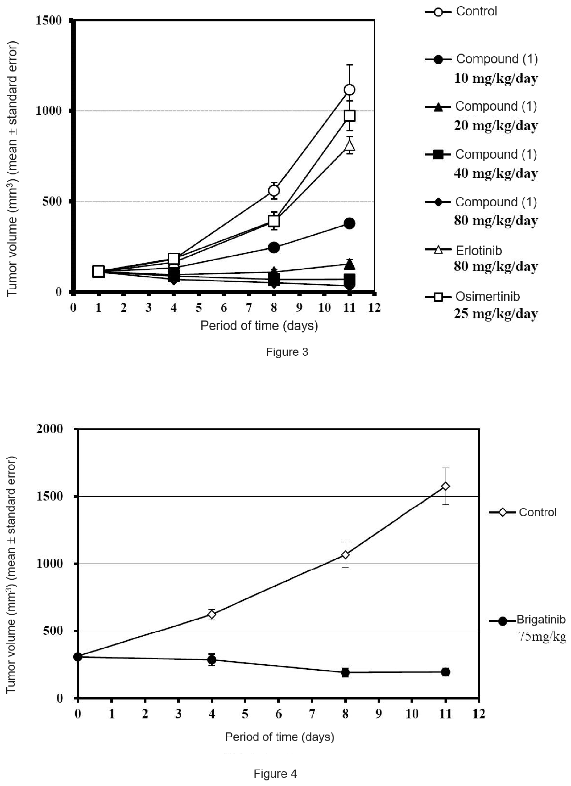

The results obtained are shown in Figure 3. In the control group, the mean tumor volume increased over time and reached 1116.1 ± 138.9 mm³ on Day 11 which was the final day of evaluation. The mean tumor volume on Day 11 of the compound (1)-receiving group was 378.5 ± 19.6 mm³, 155.1 ± 22.9 mm³, 69.7 ± 7.5 mm³ and 34.1 ± 3.9 mm³ at doses of 10, 20, 40 and 80 mg/kg/day, respectively. The mean tumor volume of the compound (1)-receiving group relative to the mean tumor volume of the control group was 33.9%, 13.9%, 6.2% and 3.1%, respectively. The tumor volume of the compound (1)-receiving group showed a statistically significant difference at all doses, relative to the control group, as analyzed by Dunnett test (p<0.001). The statistical analysis was conducted as described above.

On the other hand, in the group receiving 80 mg/kg/day erlotinib and the group receiving 25 mg/kg/day osimertinib, the mean tumor volume on Day 11 was 810.8 ± 47.1 mm³ and 972.9 ± 82.3 mm³, respectively, and the mean tumor volume of the erlotinib- and osimertinib-receiving groups relative to the mean tumor volume of the control group was 72.6% and 87.2%, respectively. Moreover, these groups both showed no statistically significant difference relative to the control group. The statistical analysis was conducted as described above.

The foregoing results indicated that in this model where erlotinib and osimertinib showed no efficacy, i.e., the mouse model transplanted subcutaneously with NIH/3T3 cells expressing EGFR with ex19del/T790M/C797S mutations, compound (1) exerted an antitumor effect in a dose-dependent manner when administered at doses from 10 mg/kg to 80 mg/kg.

### Test Example 6 Efficacy evaluation test of brigatinib in mouse subcutaneous transplantation model

The same procedure as shown in Test Example 5 was repeated to verify the effect of brigatinib on tumor growth in a BALB/cAJcl-nu/nu mouse model transplanted subcutaneously with NIH/3T3 cells expressing EGFR with Ex19del (Del E746-A750), T790M and C797S mutations (ex19del/T790M/C797S) (SEQ ID NO: 2).

On the day of cell transplantation, the cells were suspended at 1 × 10⁸ cells/mL in PBS and then mixed with an equal volume of Matrigel to prepare a cell suspension of 5 × 10⁷ cells/mL. After the mice were each immobilized, 0.1 mL per mouse of the cell suspension was injected into the right side of the chest using a 1-mL syringe equipped with a 25-G needle, whereby 5 × 10⁶ cells per mouse were transplanted.

In mice showing tumor engraftment, the long- and short-axis diameters of tumor were measured with an electronic caliper, and the tumor volume was determined according to the following equation. $Tumor volume \left({mm}^{3}\right) = long - axis diameter \left(mm\right) \times short - axis diameter {\left(mm\right)}^{2} / 2$

45 mice whose tumor volume was 237.995 mm³ to 382.316 mm³ (Mean ± SE: 308.5119 ± 5.6413 mm³) were randomly divided into groups of 5 mice each. The day of grouping was defined as Day 0.

Brigatinib was orally administered every day from Day 1 until Day 11. Brigatinib was dissolved into a dosing solvent prepared to contain 0.5 w/v% HPMC and 0.1 mol/L hydrochloric acid, thereby preparing a 7.5 mg/mL dosing solution. Based on the results of body weight measurement for each mouse on the day of administration, brigatinib was administered at a dose of 10 mL/kg per mouse.

To evaluate the antitumor effect of brigatinib, the tumor volumes on Days 0, 4, 8 and 11 were calculated for each mouse as described above to determine the mean tumor volume of each group. For comparison, a control group was provided to receive the dosing solvent for brigatinib, and the tumor volume was determined in the same manner. These animal tests were all conducted in accordance with the animal experiment guidelines of Taiho Pharmaceutical Co. Ltd., Japan (which went into effect in April 2013).

The results obtained are shown in Figure 4. In the control group, the mean tumor volume increased over time and reached 1575.34 ± 134.60 mm³ on Day 11. The mean tumor volume on Day 11 of the group receiving 75 mg/kg brigatinib was 195.63 ± 27.54 mm³. The mean tumor volume of the brigatinib-receiving group relative to the mean tumor volume of the control group was 13%. The tumor volume of the brigatinib-receiving group showed a statistically significant difference relative to the control group, as analyzed by Student t-test (p<0.001). The statistical analysis was conducted as described above.

The foregoing results indicated that in the mouse model transplanted subcutaneously with NIH/3T3 cells expressing EGFR with ex19del/T790M/C797S mutations, brigatinib exerted an antitumor effect when administered at a dose of 75 mg/kg. Taken together with the results of Test Example 4, this result indicates that brigatinib has an effect against subcutaneous tumor, but does not exert an effect against intracranial tumor growth as found in the compound of chemical formula (1).

### Test Example 7: Test of confirming whether Compound (1) is substrate for P-gp and BCRP

The *in vitro* substrate properties of Compound (1) for P-gp and BCRP were evaluated using Caco-2 cells. Caco-2 cells purchased from American Type Culture Collection were seeded on a porous membrane filter, and were cultured for a certain period of time. Thereafter, the substrate properties of Compound (1) was evaluated based on transcellular transport of the compound. The apparent permeability velocity (Papp (× 10⁻⁶ cm/sec)) of Compound (1) from the top (apical side) to the bottom (basal side) and that from the bottom (basal side) to the top (apical side) of the filter were each calculated according to the following formula. It is to be noted that the apparent permeability velocity is expressed as a value of mean ± standard deviation (n = 3). $Papp = \left[dQt/dt\right] / A / C 0$
dQ/dt: Permeability velocity (pmol/sec)
A: Cell surface area (cm²)
C0: Initial concentration of Compound (1) (pmol/mL)

Furthermore, the apparent permeability coefficient ratio (Papp ratio) was calculated according to the following formula: $Papp ratio = Papp \left(basal to apical\right) / Papp \left(apical to basal\right)$

The results are shown in Table 3. The apparent permeability coefficient ratio (Papp ratio) of Compound (1) was 1.2, 1.2, 0.9, and 0.8 at concentrations of 0.01, 0.1, 1, and 3 µmol/L, respectively, all of which were less than 2. From these results, it was determined that Compound (1) is not a substrate for P-gp or BCRP.

**Table 3**

| Compound | Concentration (µmol/L) | Papp (× 10⁻⁶ cm/sec) | | Papp ratio |
|---|---|---|---|---|
| | | Apical to basal | Basal to apical | |
| | 0.01 | 1.73 ± 0.17 | 2.07 ± 0.06 | 1.2 |
| Compound (1) | 0.1 | 2.38 ± 0.26 | 2.85 ± 0.04 | 1.2 |
| | 1 | 3.63 ± 0.35 | 3.32 ± 0.38 | 0.9 |
| | 3 | 5.56 ± 0.52 | 4.38 ± 0.61 | 0.8 |

It is to be noted that the contents as disclosed in the claims, specifications, and drawings of Japanese Patent Application No. 2023-210446 (filed on December 13, 2023) and Japanese Patent Application No. 2024-170941 (filed on September 30, 2024), which are priority documents of the present application, are incorporated in the present specification by reference.

## Claims

1. A brain-penetrable antitumor agent, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof as an active ingredient.

2. The brain-penetrable antitumor agent according to claim 1, for the treatment of primary or metastatic brain tumor.

3. The brain-penetrable antitumor agent according to claim 2, wherein the brain tumor has an EGFR mutation of C797X, where X represents any amino acid.

4. The brain-penetrable antitumor agent according to claim 3, wherein C797X is C797S or C797G.

5. The brain-penetrable antitumor agent according to claim 4, wherein the brain tumor further has an EGFR mutation of Ex19del.

6. The brain-penetrable antitumor agent according to claim 5, wherein the brain tumor further has an EGFR mutation of T790M.

7. The brain-penetrable antitumor agent according to any one of claims 1 to 6, for administration to a subject who has been treated with osimertinib.

8. A therapeutic agent for brain tumor, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof as an active ingredient.

9. The therapeutic agent for brain tumor according to claim 8, for the treatment of primary or metastatic brain tumor.

10. The therapeutic agent for brain tumor according to claim 9, wherein the brain tumor has an EGFR mutation of C797X, where X represents any amino acid.

11. The therapeutic agent for brain tumor according to claim 10, wherein C797X is C797S or C797G.

12. The therapeutic agent for brain tumor according to claim 11, wherein the brain tumor further has an EGFR mutation of Ex19del.

13. The therapeutic agent for brain tumor according to claim 12, wherein the brain tumor further has an EGFR mutation of T790M.

14. The therapeutic agent for brain tumor according to any one of claims 8 to 13, for administration to a subject who has been treated with osimertinib.

15. N-(4-(4-Amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof for use in the treatment of brain tumor.

16. The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to claim 15, for the treatment of primary or metastatic brain tumor.

17. The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to claim 16, wherein the brain tumor has an EGFR mutation of C797X, where X represents any amino acid.

18. The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to claim 17, wherein C797X is C797S or C797G.

19. The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to claim 18, wherein the brain tumor further has an EGFR mutation of Ex19del.

20. The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to claim 19, wherein the brain tumor further has an EGFR mutation of T790M.

21. The N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or salt thereof according to any one of claims 15 to 20, for administration to a subject who has been treated with osimertinib.

22. The use of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof for the manufacture of a medicament for the treatment of brain tumor.

23. The use of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof for the treatment of brain tumor.

24. A method for treating a subject with brain tumor, comprising administering N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof to a subject in need of treatment.

25. A pharmaceutical composition for the treatment of brain tumor, comprising N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a salt thereof and a pharmaceutically acceptable carrier.
